(19) Europäisches Patentamt

European Patent Office

Office européen des brevets

(11) Veröffentlichungsnummer: **0 263 316 B1**

(12) **EUROPÄISCHE PATENTSCHRIFT**

(45) Veröffentlichungstag der Patentschrift: **19.11.92**

(21) Anmeldenummer: **87113227.0**

(22) Anmeldetag: **10.09.87**

(51) Int. Cl.⁵: **A61C 19/04**

(54) Verfahren zur wenigstens zweidimensionalen Bestimmung der Relativbewegung zwischen einem Oberkiefer und einem Unterkiefer sowie Messanordnung zur Durchführung dieses Verfahrens.

(30) Priorität: **11.09.86 DE 3630945**
**28.10.86 DE 3636671**

(43) Veröffentlichungstag der Anmeldung:
**13.04.88 Patentblatt 88/15**

(45) Bekanntmachung des Hinweises auf die
Patenterteilung:
**19.11.92 Patentblatt 92/47**

(84) Benannte Vertragsstaaten:
**AT BE CH DE ES FR GB GR IT LI LU NL SE**

(56) Entgegenhaltungen:
**EP-A- 0 064 687**
**EP-A- 0 188 819**
**DE-A- 3 312 245**
**US-A- 2 119 823**
**US-A- 4 234 306**

(73) Patentinhaber: **Neumeyer, Stefan Dr.**
**Leminger Strasse 10**
**W-8491 Eschlkam(DE)**

(72) Erfinder: **Neumeyer, Stefan Dr.**
**Leminger Strasse 10**
**W-8491 Eschlkam(DE)**

(74) Vertreter: **Graf, Helmut, Dipl.-Ing. et al**
**Patentanwälte Wasmeier & Graf Postfach 10**
**08 26**
**W-8400 Regensburg 1(DE)**

# Beschreibung

Die Erfindung bezieht sich auf ein Verfahren gemäß Oberbegriff Patentanspruch 1 sowie auf eine Meßanordnung gemäß Oberbegriff Patentanspruch 10.

In der Zahnmedizin ist es vielfach erforderlich, die Relativbewegung zwischen dem Unterkiefer und dem Oberkiefer zu messen bzw. zu bestimmen. Hierfür wurden bereits Vorrichtungen vorgeschlagen, die im wesentlichen aus beidseitig am Schädel eines Patienten im Bereich der Kiefergelenke angeordneten Meßanordnungen mit Geberelementen bestehen. Diese bekannten Vorrichtungen haben den Nachteil, daß die Meßergebnisse systembedenklich nicht frei von Fehlern sind, das Anbringen der Geberelemente insbes. am Unterkiefer nicht unproblematisch ist und auch eine Bewegung des Unterkiefers relativ zum Oberkiefer zumindest psychologisch beeinträchtigt wird.

Bekannt ist weiterhin ein optisches Verfahren zur Bestimmung der Relativbewegung zwischen dem Kopf und dem Kiefer (US 21 19 823). Hierfür wird am Kopf im Bereich zwischen den Augen und am Kinn jeweils ein Bezugspunkt in Form eines kleinen Spiegels fixiert. Weitere Elemente können auch an den Zähnen fixiert werden. Mit mehreren opto-elektrischen Einrichtungen, nämlich elektrisch betriebenen Lämpchen werden Lichtstrahlen auf die Spiegel gerichtet, die diese Lichtstrahlen fokussiert auf einen Schirm reflektieren. In Abhängigkeit von der Relativbewegung zwischen dem Kiefer und dem Kopf beschreiben die reflektierten Lichtstrahlen auf dem Schirm unterschiedliche Kurven, die die Relativbewegung zwischen Kiefer und Kopf wiedergeben. Dieses bekannte Verfahren bzw. diese bekannte Vorrichtung ist zumindest bezüglich der Auswertung aufwendig und liefert keine genauen Ergebnisse.

Der Erfindung liegt die Aufgabe zugrunde, ein Verfahren zur dreidimensionalen Bestimmung der Relativbewegung zwischen einem Oberkiefer und einem Unterkiefer sowie eine Meßanordnung zur Durchführung dieses Verfahrens aufzuzeigen, welches bzw. welche mit einfachen Mitteln genaue Ergebnisse liefert, die ohne umfangreiche und komplizierte Auswertung erhalten werden.

Zur Lösung dieser Aufgabe ist ein Verfahren erfindungsgemäß entsprechend dem kennzeichnenden Teil des Patentanspruches 1 ausgebildet. Eine Meßanordnung zur Durchführung dieses Verfahrens ist entsprechend dem kennzeichnenden Teil des Patentanspruches 10 ausgebildet.

Bei dem erfindungsgemäßen Verfahren erfolgt die Bestimmung der Relativbewegung zwischen dem Oberkiefer und dem Unterkiefer bevorzugt dadurch, daß an der der opto-elektrischen Einrichtung zugewandten Seite dieser Kiefer wenigstens drei oder vier räumlich gegeneinander versetzte Bezugspunkte angebracht werden. Die Lage, die die Bezugspunkte an jedem Kiefer relativ zueinander aufweisen, die Lage, die die Bezugspunkte an einem Kiefer relativ zu den Bezugspunkten am anderen Kiefer besitzen, sowie die Änderungen dieser Lagen werden optisch erfaßt. Hieraus wird dann unter Verwendung eines Rechners (computergestützt) die Relativbewegung der beiden Kiefer zueinander ermittelt.

Bei der Erfindung erfolgt das optische Erfassen der Lage der Bezugspunkte zueinander sowie der Änderung dieser Lage mit Hilfe wenigstens einer Videokamera, vorzugsweise mit wenigstens einer Farb-Videokamera. In den von dieser Kamera gelieferten elektrischen Einzelbildern werden, bevorzugt nach einer Zwischenspeicherung in einem Bildspeicher, die vorhandenen Bezugspunkte und deren Lage zueinander ermittelt. Die die Lage der Bezugspunkte charakterisierenden Daten werden zur Bestimmung der Relativbewegung zwischen den Kiefern in einem Rechner nach einem vorgegebenen Programm ausgewertet.

Das Auffinden der Bezugspunkte in den elektrischen Einzelbildern kann dabei beispielsweise dadurch erfolgen, daß diese Bilder jeweils zeilen- und spaltenmäßig nach den aus dem übrigen Videosignal bzw. Bildinhalt markant hervortretenden Bezugspunkten zeilen- und spaltenmäßig abgetastet werden. Als Kriterium für das Auffinden der Bezugspunkte in den Einzelbildern eignen sich beispielsweise eine besondere Helligkeit und/oder eine besondere Farbgebung der Bezugspunkte.

Weiterbildungen der Erfindung sind Gegenstand der Unteransprüche.

Die Erfindung wird im Folgenden anhand der Figuren an einem Ausführungsbeispiel näher erläutert. Es zeigen:

Fig. 1 in schematischer Darstellung den Aufbau einer Meßanordnung zur dreidimensionalen Bestimmung der Relativbewegung zwischen dem menschlichen Oberkiefer und dem Unterkiefer in Seitenansicht unter Verwendung einer Videokamera;

Fig. 2 die an den Vorder- bzw. Schneidezähnen eines menschlichen Ober- bzw. Unterkiefers befestigten Bezugselemente der Meßanordung gemäß Fig. 1 in Blickrichtung des Pfeiles A der Fig. 1.;

Fig. 3 in schematischer Darstellung eine Meßanordnung unter Verwendung von zwei Videokameras;

Fig. 4 in schematischer Darstellung eine Meßanordnung unter Verwendung einer Lasereinrichtung;

Fig. 5     in schematischer Darstellung eine Meßanordnung unter Verwendung von zwei Lasereinrichtungen;

Fig. 6     in schematischer Darstellung eine weitere Ausführungsform der Meßanordnung mit Lasereinrichtung;

Fig. 7 bis 9     in schematischer Darstellung weitere Ausführungsformen der Meßanordnung.

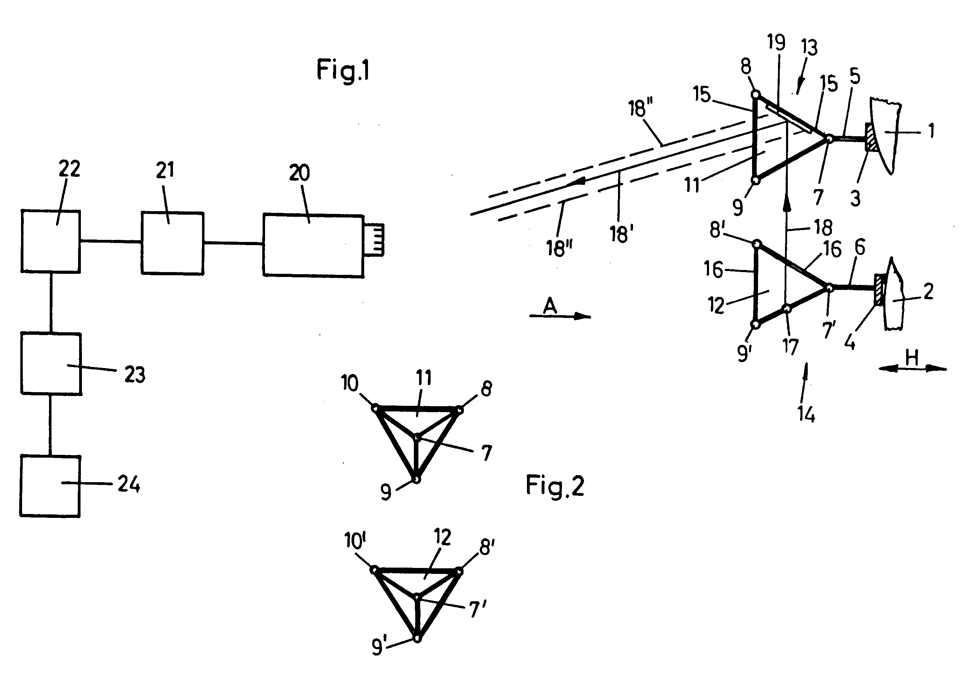

Die in der Fig. 1 dargestellte Meßanordnung dient zur dreidimensionalen Messung der Relativbewegung zwischen dem Oberkiefer und dem Unterkiefer eines Patienten, wobei der einfacheren Darstellung wegen von dem Oberkiefer und dem Unterkiefer jeweils nur ein einziger Schneidezahn 1 bzw. 2 wiedergegeben sind. An der Vorderseite der vorhandenen Schneidezähne 1 des Oberkiefers ist ein streifenförmiges, gekrümmtes Halteelement 3, vorzugsweise ein solches aus thermoplastischem Material durch Kleben oder auf andere geeignete Weise befestigt. Ein ähnlich ausgebildetes Halteelement 4 ist an der Vorderseite der am Unterkiefer vorhandenen Schneidezähne 2 befestigt. An der den Schneidezähnen 1 bzw. 2 abgewendeten Oberflächenseite trägt jedes Halteelement 3 bzw. 4 ein stabförmiges Element 5 bzw. 6, welches mit seinem freien Ende über das betreffende Halteelement wegsteht, mit seiner Längserstreckung quer zu den Oberflächenseiten des betreffenden Halteelementes liegt und an seinem freien Ende mit dem einen Eckpunkt 7 bzw. 7′ eines insgesamt vier Eckpunkte 7, 8, 9 und 10 bzw. 7′, 8′, 9′ und 10′ aufweisenden pyramidenartigen Körpers 11 bzw. 12 verbunden ist. Der pyramidenartige Körper 11 mit den Eckpunkten 7 - 10 bildet zusammen mit dem stabförmigen Element 5 und dem Halteelement 3 das eine Bezugselement 13 udn der pyramidenartige Körper 12 mit den Eckpunkten 7′ - 10′ zusammen mit dem stabförmigen Element 6 und dem Halteelement 4 das andere Bezugselement 14 der Meßanordnung. Beide Bezugselemente 13 und 14 sind so ausgebildet, daß aufgrund der stabförmigen Elemente 5 bzw. 6 die pyramidenartige Körper 11 bzw. 12 mit allen ihren Eckpunkten außerhalb der Mundhöhle vor den Lippen angeordnet sind, die Eckpunkte 7 bzw. 7′ einen kleineren Abstand von den Schneidezähnen 1 bzw. 2 und die Eckpunkte 8, 9 und 10 bzw. 8′, 9′ und 10′ jeweils einen größeren Abstand von den Schneidezähnen 1 bzw. 2 aufweisen, wobei bei der dargestellten Ausführungsform weiterhin die Achse der stabförmigen Elemente 5 bzw. 6 die dem Eckpunkt 7 bzw. 7′ gegenüberliegende, von den Eckpunkten 8, 9 und 10 bzw. 8′, 9′ und 10′ begrenzte dreieckförmige Fläche unter einem Winkel von etwa 90° schneidet. Die pyramidenartigen Körper 11 und 12 sind weiterhin so angeordnet, daß die von den Eckpunkten 8 - 10 bzw. 8′ - 10′ begrenzte dreieckförmige Fläche jeweils eine obere, im wesentlichen horizontale und zwischen den Eckpunkten 8 und 10 bzw. 8′ und 10′ liegende Seitenlänge mit unterhalb dieser Seitenlänge angeordnetem Eckpunkt 9 bzw. 9′ aufweist. Die pyramidenartigen Körper 11 und 12 bestehen aus an den Eckpunkten 7 - 10 bzw. 7′ - 10′ miteinander verbundenen, bei der dargestellten Ausführungsform jeweils gleichlangen stabförmigen Elementen 15 bzw. 16, so daß auch der jeweils in Betrachtungsrichtung (Pfeil A) hintere Eckpunkt 7 bzw. 7′ voll sichtbar ist. An dem die Eckpunkte 7′ und 9′ miteinander verbindenden stabförmigen Element 16 des Bezugselementes 14 ist eine Lichtquelle 17 befestigt, die einen gebündelten, in vertikaler Richtung nach oben gerichteten Lichtstrahl 18 aussendet, der in der dargestellten Meßanordnung auf einen in der Ebene der Eckpunkte 7, 8 und 10 des Bezugselementes 13 angeordneten bzw. an den dortigen stabförmigen Elementen 15 befestigten Spiegel 19 auftrifft und an diesem Spiegel nach vorne reflektiert wird, wie dies durch den reflektierten Lichtstrahl 18′ angedeutet ist. Da der Spiegel 19 gegenüber einer horizontalen Achse geneigt ist, in deren Richtung bei der dargestellten Ausführungsform in etwa auch die Achsen der stabförmigen Elemente 5 und 6 liegen, verschiebt sich bei einer horizontalen Bewegung des Unterkiefers relativ zum Oberkiefer in dieser horizontalen Achsrichtung (Doppelpfeil H) der reflektierte Lichtstrahl nach oben bzw. unten, wie dies in der Fig. 1 mit den unterbrochenen Linien 18″ angedeutet ist, wobei diese Verschiebung ein Maß für die Relativbewegung des Unterkiefers zum Oberkiefer in Richtung des Doppelpfeiles H ist.

Die Eckpunkte 7 - 10 bzw. 7′ - 10′ sind gegenüber den übrigen Teilen der Bezugselemente 13 bzw. 14 im Kontrast bzw. in der Helligkeit und/oder in der Farbgebung markant ausgeführt und weisen bevorzugt eine Farbgebung auf, die von der Gesichtsfarbe, aber auch von der Farbe der Lippen markant abweicht.

Die Meßanordnung besteht weiterhin aus einer Videokamera 20, die in einem vorgegebenen Abstand von dem beispielsweise auf einem Stuhl sitzenden Patienten angeordnet und mit ihrem Objektiv in Betrachtungsrichtung (Pfeil A) auf den Mund des Patienten bzw. auf die an den Schneidezähnen 1 und 2 befestigten Bezugselemente 13 und 14 gerichtet ist. Mit der Videokamera wird die bei einer Relativbewegung zwischen dem Unterkiefer und dem Oberkiefer (Kaubewegungen usw.) auftretende Bewegung der Bezugselemente 13 und 14 bzw. die Verlagerung der Eckpunkte 7 - 10 und 7′ - 10′ sowie die Lageänderung des reflektierten Licht-

strahles 18' aufgenommen. Die entsprechenden Bildsignale werden in einem Bildspeicher 21 gespeichert, der beispielsweise zumindest teilweise von einem Video-Recorder gebildet ist. Mit Hilfe einer elektronischen Schaltung 22 können dann die im Bildspeicher 21 gespeicherten Einzelbilder zeilen- und spaltenmäßig abgetastet werden, wobei immer dann ein Signal an einen Datenspeicher 23 abgegeben wird, wenn bei dieser Abtastung einer der markant ausgebildeten Eckpunkte 7 - 10 bw. 7' - 10' oder der durch seine Helligkeit besonders markante reflektierte Lichtstrahl 18' ermittelt wird. Aus der jeweiligen Abtastphase ergibt sich dann auch die Lage des jeweils ermittelten Eckpunktes bzw. des reflektierten Lichtstrahles 18', so daß die diese Lage charakterisierenden elektrischen Signale bzw. Daten in dem Datenspeicher 23 gespeichert werden können. Die im Datenspeicher 23 gespeicherten Daten können dann einem Rechner 24 zugeführt werden, welcher aus diesen Daten nach einem geeigneten Programm die Relativbewegung zwischen dem Oberkiefer und dem Unterkiefer ermittelt. Die so gewonnenen Daten können dann den verschiedenen weiteren Zwecken zugeführt werden bzw. für die unterschiedlichsten Anwendungen verwendet werden, beispielsweise zur optischen Anzeige der Relativbewegung zwischen Unterkiefer und Oberkiefer, zum Ausdrucken des zeitlichen Verlaufs der verschiedenen Bewegungskomponenten dieser Relativbewegung usw. Weiterhin können diese Daten auch gespeichert und später zum Vergleich mit der in einem zahnärztlichen Artikulator simulierten Relativbewegung zwischen Unterkiefer und Oberkiefer verwendet werden.

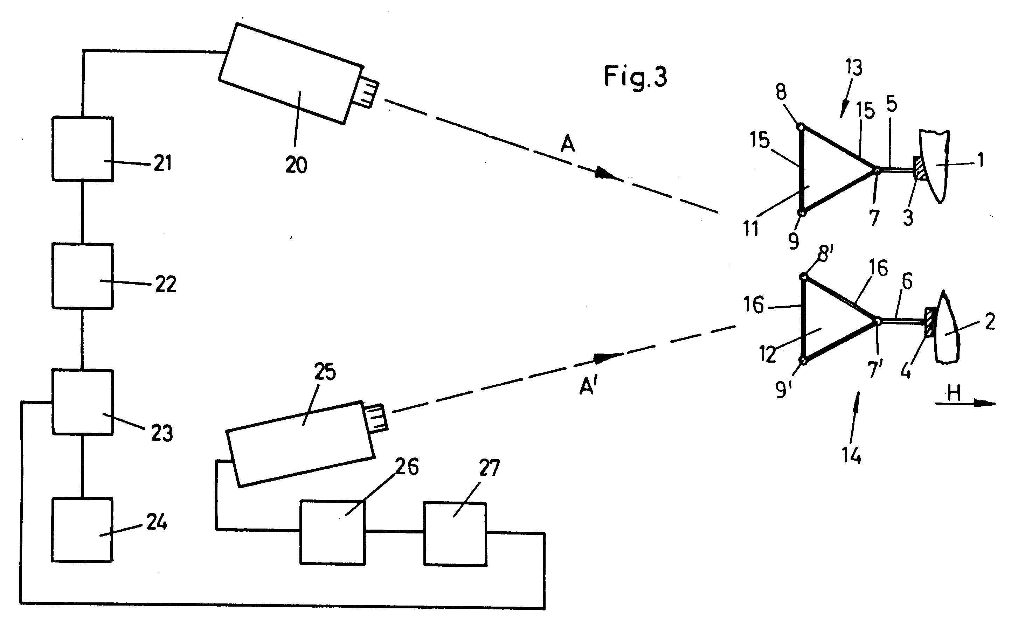

In der Fig. 3 ist eine weitere Meßanordnung dargestellt, die sich von der Meßanordnung nach Fig. 1 im wesentlichen dadurch unterscheidet, daß zusätzlich zu der Videokamera 20 eine weitere Videokamera 25 mit zugehörigem Bildspeicher 26 und zugehöriger elektronischer Schaltung 27 vorgesehen ist. Der Bildspeicher 26 sowie die elektronische Schaltung 27 entsprechen in ihrer Funktion dem Bildspeicher 21 und der elektronischen Schaltung 22. Die Videokamera 25 ist in gleicher Weise wie die Videokamera 20 in einem vorgegebenen Abstand von den beispielsweise auf einen Stuhl sitzenden Patienten angeordnet und mit ihrem Objektiv in Betrachtungsrichtung (Pfeil A') auf den Mund des Patienten bzw. auf die an den Schneidezähnen 1 und 2 befestigten Bezugselemente 13 und 14 gerichtet, wobei allerdings bei der in der Fig. 3 dargestellten Ausführungsform die optischen Achsen (Pfeile A und A') der beiden Videokameras 20 und 25 einen Winkel von 45° miteinander einschließen, der sich zu der die Videokameras 20 und 25 aufweisenden Seite der Meßanordnung, d.h. bei der für die Fig. 3 gewählten Darstellung zur linken Seite hin öffnet. Die von der Videokamera 25 gelieferten Bildsignale werden in dem Bildspeicher 26 gespeichert, der beispielsweise wiederum zumindest teilweise von einem Video-Rekorder gebildet ist. Selbstverständlich kann für den Bildspeicher 26 ebenso wie für den Bildspeicher 21 ein anderer, geeigneter Speicher eingesetzt werden. Mit Hilfe der elektronischen Schaltung 27 können dann die im Bildspeicher 26 gespeicherten Einzelbilder zeilen- und spaltenmäßig abgetastet werden, wobei immer dann ein Signal an den mit der elektronischen Schaltung 27 ebenfalls verbundenen Datenspeicher 23 abgegeben wird, wenn bei dieser Abtastung einer der markant ausgebildeten und die Bezugspunkte bei der Messung bildenden Eckpunkte 7 - 10 bzw. 7' - 10' ermittelt wird. Aus der jeweiligen Abtastphase ergibt sich dann auch die Lage des jeweils von der Videokamera 25 ermittelten Eckpunktes, so daß diese Lage zusammen mit der Lage der von der Videokamera 20 ermittelten Eckpunkte im Datenspeicher 23 gespeichert werden kann. Die im Datenspeicher gespeicherten Daten werden dann dem Rechner 24 zugeführt, welche aus diesen Daten wiederum nach einem geeigneten Programm die Lage, die die Eckpunkte 7 - 10 bzw. 7' -10' an jedem Bezugselement 13 bzw. 14 relativ zueinander aufweisen sowie auch die Lage ermittelt, die die einzelnen Bezugspunkte 7 - 10 des Bezugselementes 13 relativ zu den Bezugspunkten 7'- 10' des Bezugselementes 14 besitzen, womit dann auch die Relativbewegung zwischen dem Oberkiefer und dem Unterkiefer ermittelt ist. Die in der Fig. 3 dargestellte Ausführung hat den Vorteil, daß für die Bewegung in Richtung des Doppelpfeiles H die Lichtquelle 17 und der Spiegel 19 nicht erforderlich sind, sondern die die Bewegungsrichtung in dieser horizontalen Achse durch die gegeneinander geneigten optischen Achsen der Videokameras 20 und 25 ermittelt werden kann.

Selbstverständlich ist es bei der in der Fig. 3 dargestellten Ausführungsform auch möglich, die beiden Videokameras 20 und 25 an einen einzigen, zwei Kanäle oder Speicher aufweisenden Bildspeicher anzuschließen, dessen Ausgangssignal dann mit einer einzigen elektronischen Schaltung im Multiplex-Verfahren ausgewertet wird, und zwar in einer ersten Zeitperiode zunächst das Bildsignal der Videokamera 20 und in einer darauffolgenden Zeitperiode das Bildsignal der Videokamera 25. Unabhängig hiervon kann selbstverständlich sowohl bei der Ausführung nach Fig. 1, als auch bei der Ausführung nach Fig. 3 auf die Bildspeicher 21 bzw. 26 verzichtet werden, wobei dann das von der Videokamera 20 bzw. 25 gelieferte Signal jeweils sofort in einer entsprechenden elektronischen Schaltung 22 bzw. 27 in der oben beschriebenen Weise ausgewertet wird. Unabhängig hiervon ist es weiterhin auch möglich die Auswertung der von den Videokameras 20 bzw. 25 gelieferten Signale bzw. die Bestimmung der Lage der Eckpunkte 7 - 10 bzw. 7′ - 10′ in der Weise vorzunehmen, daß die in den Videobildern der Videokameras 20 bzw. 25 wiedergegebene Lage der Eckpunkte 7 - 10 bzw. 7′ - 10′ mit einem vorgegebenen optischen oder elektronischen Raster verglichen wird und aus diesem Vergleich dann die tatsächliche Lage der genannten Eckpunkte ermittelt wird. Dies kann beispielsweise dadurch geschehen, daß in der elektronischen Schaltung 22 bzw. 27 ein derartiges elektronisches Raster erzeugt wird, so daß dann beim zeilen-und spaltenmäßigen Abtasten bzw. Erfassen des entsprechenden Video-Signals der beim Abtasten auftretende Abstand des einem Eckpunkt 7 - 10 bzw. 7′ - 10′ entsprechenden Signals von einem vorausgegangenen oder nachfolgenden Rastersignal zeilen- und spaltenmäßig erfaßt und hieraus unter Bezugnahme auf dieses, eine bestimmte Lage definierende Rastersignal die tatsächliche Lage des betreffenden Eckpunktes 7 - 10 bzw. 7′ - 10′ ermittelt wird. Insbesondere bei einer entsprechend feinen Rastereinteilung läßt sich auf diese Weise eine besonders hohe Genauigkeit bei der Ermittlung der tatsächlichen Lage der Eckpunkte 7 - 10 bzw. 7′ -10′ erreichen.

Die in der Fig. 3 dargestellte Meßanordnung, bei der die Bezugselemente 13 und 14 unter zwei, einen Winkel mit einander einschließenden Betrachtungsrichtungen (A, A′) betrachtet wird, läßt sich auch mit einer einzigen Video-Kamera dadurch realisieren, daß die Bezugspunkte mit dieser Kamera über eine Spiegeleinrichtung abwechselnd jeweils aus der einen Betrachtungsrichtung und anschließend aus der anderen Betrachtungsrichtung betrachtet werden, so daß dann in zeitlicher Folge aufeinander dem Bildspeicher bzw. der elektronischen Schaltung zur Auswertung Video-Signale solche Signale aus den beiden Betrachtungsrichtungen zugeführt werden.

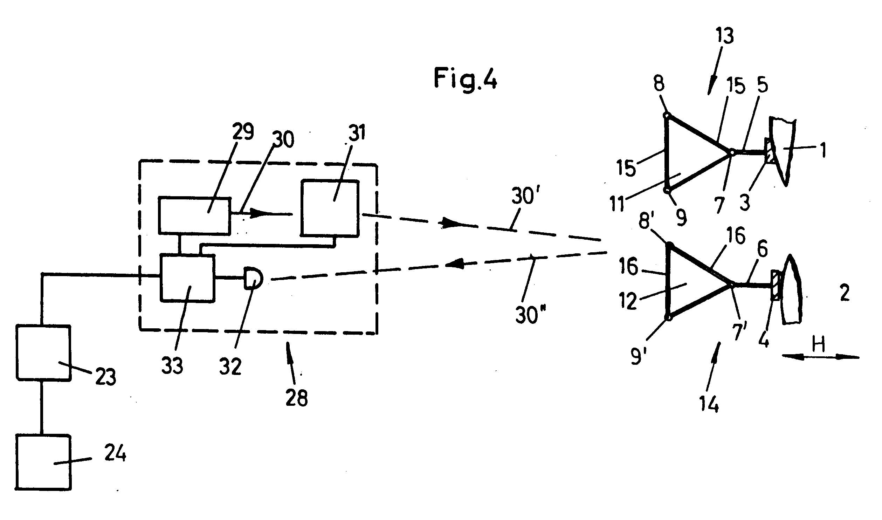

Fig. 4 zeigt eine Ausführung, bei der die Bestimmung der Lage der Eckpunkte 7 - 10 bzw. 7′ - 10′ nicht mit Hilfe einer oder mehrerer Videokameras, sondern mit Hilfe einer Laser-Einrichtung 28 erfolgt. Diese Einrichtung besteht aus einem Laser 29, der einen punktförmigen bzw. stark gebündelten Lichtstrahl 30 einer vorgegebenen Wellenlänge aussendet, aus einer beispielsweise zwei Ablenkspiegel aufweisenden Umlenk- oder Abtasteinrichtung 31 für den Lichtstrahl 30, aus einem Lichtempfänger 32 sowie aus einer elektronischen Meß- und Auswertungeinrichtung 33, deren Ausgang wiederum mit dem Datenspeicher 23 für den Rechner 24 verbunden ist. Die Lasereinrichtung 28 ist in einem vorgegebenen Abstand von den beispielsweise auf einen Stuhl sitzenden Patienten so angeordnet, daß der mit der Umlenkeinrichtung abgelenkte Lichtstrahl 30′ im zwei senkrecht zueinander verlaufenden Achsrichtungen, beispielsweise in der vertikalen Achse und in der senkrecht zur Zeichenebene verlaufenden transversalen Achse den von den Eckpunkten 7 - 10 und 7′ - 10′ der Bezugselemente 13 und 14 gebildeten Meßbereich zeilen-und spaltenmäßig abtastet. Der dabei an den Lichtempfänger 32 reflektierte Lichtstrahl 30″ wird in diesem Empfänger in ein elektrisches Signal umgewandelt, welches der Meß- und Auswertungseinrichtung 33 zugeführt wird, und zwar zusammen mit einem von der Umlenkeinrichtung 31 abgeleiteten Signal, welch letzteres den jeweiligen räumlichen Winkel berücksichtigt, den der Lichtstrahl 30′ beispielsweise zu einer Bezugsachse aufweist. Aus diesen Signalen sowie ggf. aus einen weiteren, vom Laser 29 oder dessen, nicht näher dargestellte Ansteuereinrichtung abgeleiteten Signal wird dann in der Meß- und Auswertungseinrichtung (aufgrund des jeweiligen Ablenkwinkels des Lichtstrahles 30′ sowie aus der Entfernung, die die Eckpunkte 7 - 10 bzw. 7′ - 10′ von der Lasereinrichtung 28 in der horizontalen Achsrichtung H aufweisen) die tatsächliche Lage dieser Eckpunkte ermittelt und die entsprechenden Werte werden von der Meß- und Auswertungseinrichtung an den Datenspeicher 23 weitergegeben. Die für die Ermittlung der Lage der Eckpunkte notwendige Entfernungsmessung wird bei der dargestellten Meßanordnung in der gleichen Weise durchgeführt, wie dies bei entsprechenden, mit einem Laser-Lichtstrahl arbeitenden Entfernungs-Meßeinrichtungen üblich ist. Für diese Entfernungsmessung kann beispielsweise die Laufzeit zwischen dem ausgesandten Lichtstrahl 30 und dem auf den Empfänger 32 auftretenden Lichtstrahl bei einem im Impulsbetrieb arbeitenden Laser 29 verwendet werden. Zur Ermittlung der Entfernung kann auch eine Winkelmessung zwischen den an den Bezugselementen 13 und 14 jeweils in einem vorgegebenen Abstand zu einander angeordneten Eckpunkten 7 - 10 bzw. 7′ - 10′ dienen. Weiterhin ist auch eine Interferenzmessung zur Bestimmung der Entfernung möglich.

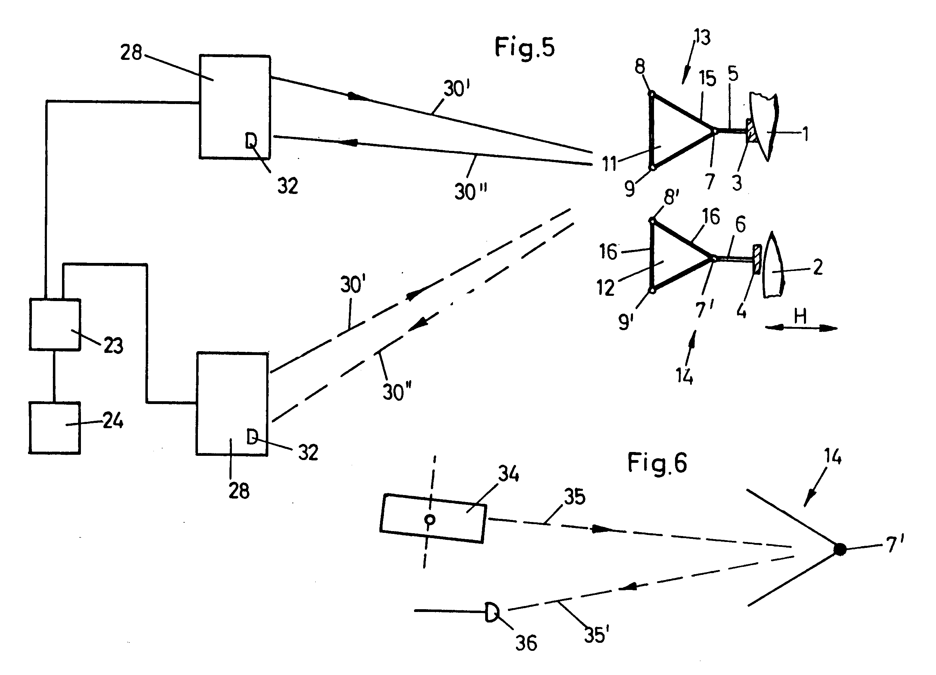

Fig. 5 zeigt in vereinfachter Darstellung eine weitere Ausgestaltung der Meßeinrichtung, die sich von der Ausführung nach Fig. 4 dadurch unterscheidet, daß zwei Laser-Einrichtungen 28 vorgesehen sind, die jeweils den Meßbereich, d.h. die Eckpunkte 7 - 10 bzw. 7′ - 10′ mit einem Lichtstrahl 30′, vorzugweise jedoch mit unterschiedlicher Wellenlänge synchron abtasten. Die beiden Lasereinrichtungen 28 sind in räumlichem Abstand voneinander angeordnet, so daß die von diesen Lasereinrichtungen ausgehenden abtastenden Lichtstrahlen 30′ jeweils einen Winkel miteinander einschließen und somit aus den von den Meß-und Auswertungseinrichtungen 33 der Lasereinrichtungen 28 gelieferten Signale auch die jeweilige Lage der Eckpunkte 7 - 10 bzw. 7′ - 10′ bzw. die Änderung dieser Lage in der horizontalen Achsrichtung ermittelt werden kann, ohne daß die oben im Zusammenhang mit der Fig. 4 beschriebene Entfernungsmessung durch die Lasereinrichtung 28 notwendig ist.

Das Schwenken bzw. Bewegen des abtastenden Lichtstrahles 30′ kann bei den Ausführungsformen nach Fig. 4 und 5 selbstverständlich auch dadurch erreicht werden, daß der Laser 29 oder ein diesen Laser aufweisender Teil der jeweiligen Lasereinrichtung 28 um zwei senkrecht zueinander verlaufende Raumachsen geschwenkt werden.

Weiterhin ist es bei der in der Fig. 5 dargestellten Ausführung grundsätzlich auch möglich, die beiden abtastenden Lichtstrahlen 30′ dadurch zu erreichen, daß ein von einen einzigen Laser 29 ausgesandter Lichtstrahl beispielsweise unter Verwendung eines halbdurchlässigen Spiegels in die beiden Lichtstrahlen 30′ aufgeteilt wird, wobei dann in diesem Fall die beiden Lasereinrichtungen 28 zumindest teilweise eine gemeinsame Einrichtung bilden.

In der Fig. 6 ist in vereinfachter Darstellung eine weitere Ausführungsform einer mit einem Laserstrahl arbeitenden Meßeinrichtung dargestellt. Diese besitzt einen Laser 34, der wiederum im Abstand von dem Meßbereich, d.h. von dem Bezugselementen 13 und 14 angeordnet ist und einen gebündelten Lichtstrahl 35 aussendet. Die Funktionsweise dieser Meßanordnung beruht im wesentlichen darauf, daß einer der beiden Körper, deren Relativbewegung gemessen werden soll, feststeht und sich nur der andere Körper bewegt. Der Lichtstrahl 35 wird bei dieser Meßanordnung auf einen markanten Bezugspunkt des sich bewegenden Körpers, d.h. beispielsweise auf den Eckpunkt 7′ des am Unterkiefer befestigten Bezugselementes 14 ausgerichtet. Letzteres erfolgt entweder durch manuelle Einstellung des Lasers 34, oder aber dadurch, daß dieser Laser zunächst zur Erzielung einer reihen- und spaltenmäßigen Abtastbewegung des Lichtstrahles 35 geschwenkt wird, und dann, wenn ein Lichtstrahl 35′ von dem markanten Bezugspunkt, d.h. von dem Eckpunkt 7′ an den Lichtempfänger 36 reflektiert wird, durch ein von diesem Lichtempfänger erzeugtes Signal die Schwenkbewegung des Lasers 34 unterbrochen wird, wobei der Laser 34 dann zunächst einmal die zuletzt eingenommene Stellung beibehält. Beim Bewegen des Eckpunktes 7′ wird der Laser 34 dann so mitgeführt bzw. geschwenkt, daß der Lichtstrahl 35 ständig auf den Eckpunkt 7′ auftrifft, so daß aus dieser Schwenkbewegung, die der Laser 34 beim Mitführen ausführt, die Bewegung des Eckpunktes 7′ in der vertikalen und transversalen Achsrichtung, d.h. in der Ebene senkrecht zur Zeichenebene der Fig. 6 sehr genau ermittelt werden kann. Als Steuerkriterium für das Mitführen des Lasers 34 dient der auf den Empfänger 36 auftreffende Lichtstrahl 35′, d.h. durch eine von dem Empfänger 36 angesteuerte Regel- und Steuereinrichtung wird der Laser 34 jeweils so nachgeführt, daß der auf den Empfänger 36 reflektierte Lichtstrahl 35′ am Ausgang dieses Empfängers ein maximales Signal erzeugt. Um eine Richtungsvorgabe bei dem Nachführen des Lasers 34 zu erreichen, weist die Meßeinrichtung auch Mittel auf, die die Richtung der Bewegung des Eckpunktes 7′ in der jeweiligen Achsrichtung feststellt. Dies ist beispielsweise durch Verwendung einer Photodiode mit Leuchtfeldbegrenzung oder aber dadurch möglich, daß der Laser 34 zusätzlich zu der Nachführ-Bewegung auch eine oszillierende Bewegung um die beiden Achsrichtungen ausführt, um die der Laser 34 beim Nachführen geschwenkt wird, so daß dann aus dem Zeitpunkt, in welchem bei dieser oszillierenden Bewegung das Maximum des von dem Empfänger 36 gelieferten Signals auftritt, die Richtung der Bewegung des Eckpunktes 7′ in den beiden Achsrichtungen ermittelt werden kann.

In Verbindung mit einer Entfernungsmeßeinrichtung kann mit der Meßeinrichtung nach Fig. 6 auch die Bewegung des Eckpunktes 7′ in der horizontalen Achsrichtung festgestellt werden. Ohne diese Entfernungsmeßeinrichtung ist die Meßanordnung der Fig. 6 dann brauchbar, wenn eine Bewegung des Bezugspunktes bzw. Eckpunktes 7′ nur in einer Ebene zu erwarten ist. Die Meßeinrichtung nach Fig. 6 kann aber auch mit anderen, vorbeschriebenen Meßeinrichtungen kombiniert werden.

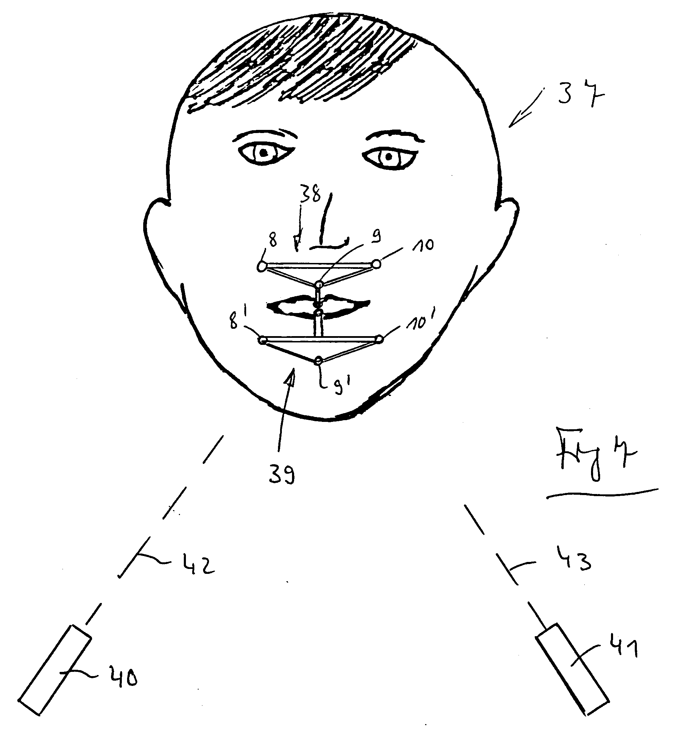

Eine weitere Ausführungsform ist in der Fig. 7 dargestellt. Bei dieser Ausführungsform sind am Ober- und Unterkiefer des Kopfes 37 eines Patienten jeweils nur die drei Bezugspunkte 8 - 10 bzw. 8′ - 10′ vorgesehen, die wiederum räumlich gegeneinander versetzt und die Eckpunkte eines Dreieckes bilden. Die Bezugspunkte 8 - 10 bzw. 8′ -10′ sind an jeweils einem Bezugselement 38 bzw. 39 gebildet, wobei jedes Bezugselement zum Festlegen am Oberkiefer bzw. am Unterkiefer bzw. an den dortigen Zahnreihen an einer Bißgabel vorgesehen ist. Um eine dreidimensionale Bestimmung der Relativbewegung zwischen Oberkiefer und Unterkiefer zu ermöglichen, sind bei dieser Ausführungsform zwei opto-elektronische Einrichtungen 40 und 41 vorgesehen, die räumlich versetzt so angeordnet sind, daß sie den von den Bezugspunkten 8 - 10 und 8′ - 10′ gebildeten Meßbereich aus zwei unterschiedlichen Achsrichtungen (Betrachtungsachsen) erfassen.

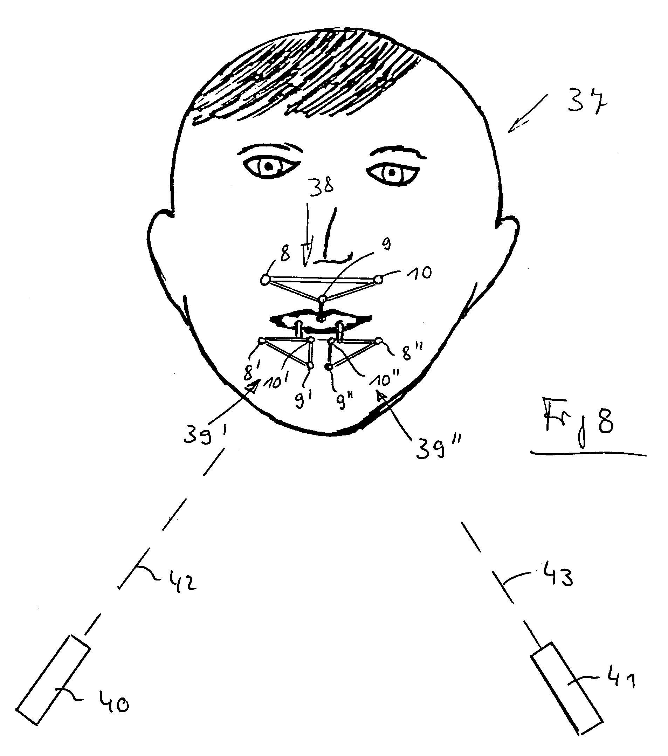

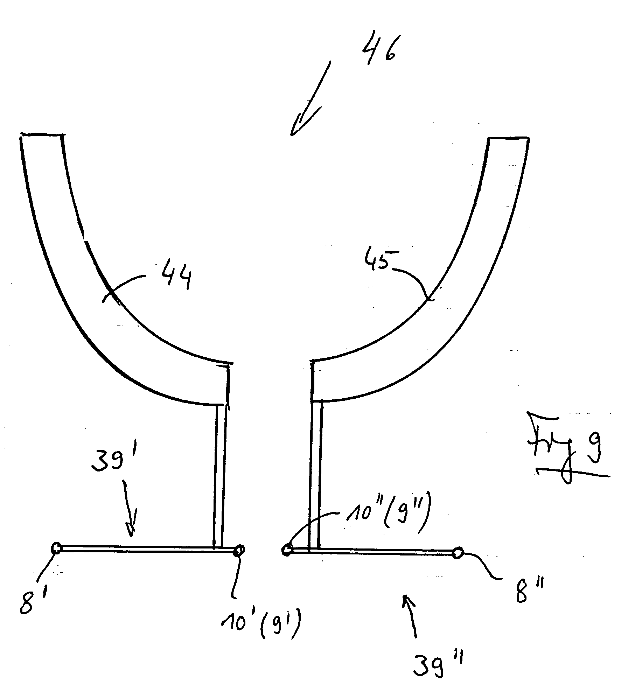

Fig. 8 zeigt eine ähnliche Ausführung wie Fig. 7, wobei bei der Ausführung nach Fig. 8 am Unterkiefer jedoch zwei Paare von jeweils drei Bezugspunkten, nämlich von den Bezugspunkten 8′ -10′ und den Bezugspunkten 8″ - 10″ gebildet sind, wobei die Bezugspunkte 8′ - 10′ bzw. 8″ - 10″ beider Paare jeweils räumlich gegeneinander so versetzt sind, daß sie wiederum die Eckpunkte eines Dreieckes bilden, wobei die beiden Dreiecke spiegelbildlich zu einer vertikalen und senkrecht zur Vorderseite des Kopfes 37 verlaufenden Mittelebene M angeordnet sind und die Bezugspunkte 10' und 9' bzw. 10'' und 9'', die die der Mittelebene M benachbarten Seiten der Dreiecke bilden, jeweils in vertikaler Richtung übereinander vorgesehen sind. Die Bezugspunkte 8' - 10' sind an einem Bezugselement 39' und die Bezugspunkte 8'' -10'' an einem Bezugselement 39'' vorgesehen. Beide Bezugselemente 39' und 39'' sind getrennt am Unterkiefer befestigt, und zwar beispielsweise mit Hilfe der Teile 44 und 45 einer zweiteiligen Bißgabel 46, wobei die Bezugspunkte 8' -10' am Teil 44 und die Bezugspunkte 8'' - 10'' am Teil 45 vorgesehen sind. Diese Ausgestaltung hat den Vorteil, daß mit den Einrichtungen 40 und 41 nicht nur eine dreidimensionale Erfassung bzw. Messung der Relativbewegung zwischen dem Oberkiefer und dem Unterkiefer möglich ist, sondern mit diesen Einrichtungen auch beim Bewegen des Unterkiefers bzw. beim Kauen auftretende Deformationen des Unterkiefers erfaßt werden können. Bei der in den Figuren 8 und 9 dargestellten Ausführung sind am Unterkiefer insgesamt sechs Bezugspunkte 8' - 10' und 8'' -10'' vorgesehen. Grundsätzlich wären jedoch insgesamt fünf Bezugspunkte ausreichend. Soll die Deformation des Oberkiefers erfaßt werden, so sind die Bezugspunkte 8 - 10 am Unterkiefer und die Bezugspunkte 8' -10' sowie 8'' - 10'' am Oberkiefer vorgesehen.

Bei den in den Figuren 7 und 8 dargestellten Ausführungsformen sind die Bezugspunkte 8 - 10, 8' - 10' bzw. 8'' - 10'' jeweils so angeordnet, daß die von den Bezugspunkten gebildeten Dreiecke mit ihren Flächen den Einrichtungen 40 und 41 zugewendet sind, die Betrachtungsachsen 42 und 43 dieser mit Abstand von der Vorderseite des Kopfes 37 angeordneten Einrichtungen also einen Winkel mit der Fläche dieser Dreiecke einschließen.

Die Erfindung wurde voranstehend an Ausführungsbeispielen erläutert. Es versteht sich, daß Änderungen sowie Abwandlungen möglich sind, ohne daß dadurch der der Erfindung zugrundeliegende Erfindungsgedanke verlassen wird. So ist es beispielsweise auch möglich, anstelle der von den Eckpunkten 7 - 10 bzw. 7' -10' an den Bezugselementen 13 und 14 gebildeten Bezugspunkte wenigstens an einem der beiden Körper, beispielsweise am Kopf bzw. Schädel des Patienten solche zu verwenden, die dort direkt angebracht sind. Die bei der Erfindung verwendeten Lichtempfänger 32 und/oder 36 sind Photodioden oder Photodiodenanordnungen oder andere geeignete photo-elektrische Wandler, die beim Auftreffen von Licht ein elektrisches Signal erzeugen, wie z.B. Phototransistoren oder Photowiderstände.

Zur Erleichterung des Auffindens und/oder zur Identifikation der Bezugspunkte bei der Bestimmung der Relativbewegung können diese Bezugspunkte auch von lichtemittierenden Elementen (z.B. Leuchtdioden) gebildet sein, die dann z.B. jeweils Licht unterschiedlicher Farbgebung und/oder Helligkeit bzw. Wellenlänge und/oder moduliertes Licht, d.h. in der Intensität moduliertes Licht aussenden, wobei es hierbei für die Identifikation der einzelnen Bezugspunkte vorteilhaft sein kann, daß das von wenigstens einem Teil der Bezugspunkte ausgesandte Licht sich in der Modulationsfrequenz von dem Licht der anderen Bezugspunkte unterscheidet.

Selbstverständlich können als Bezugspunkte auch an Bezugselementen oder -körpern gebildete markante Stellen oder Linien verwendet werden.

**Patentansprüche**

1. Verfahren zur dreidimensionalen Bestimmung der Relativbewegung zwischen einem Unterkiefer und einem Oberkiefer, wobei an den Kiefern (1, 2) Bezugspunkte (7 - 10, 7' -10'; 8'' - 10'') angebracht werden, deren Lage relativ zueinander in wenigstens einer Achsrichtung optisch erfaßt wird, und wobei aus der Lageänderung der Bezugspunkte zueinander die Relativbewegung von Unterkiefer (2) und Oberkiefer (1) ermittelt wird, **dadurch gekennzeichnet, daß** am Unterkiefer (2) und am Oberkiefer (1) jeweils wenigstens drei räumlich gegeneinander versetzte Bezugspunkte (7 - 10, 7' - 10'; 8'' - 10'') angebracht werden, daß die Lage der Bezugspunkte (7 - 10, 7' - 10'; 8'' -10'') zueinander mit Hilfe wenigstens einer Videokamera (20, 25) erfaßt und hieraus computergestützt die Relativbewegung von Unterkiefer und Oberkiefer ermittelt wird.

2. Verfahren nach Anspruch 1, dadurch gekennzeichnet, daß die Lage der Bezugspunkte (7 - 10, 7' - 10'; 8'' - 10'') zueinander mit Hilfe eines an die Videokamera angeschlossenen Bildspeichers (21) erfaßt wird.

3. Verfahren nach Anspruch 1 oder 2, dadurch gekennzeichnet, daß die von der Videokamera (20, 25) gelieferten und/oder im Bildspeicher (21, 26) gespeicherten Einzelbilder zur Bestimmung der Lage der Bezugspunkte (7 - 10, 7' - 10; 8'' - 10'') zeilen- und spaltenmäßig abgetastet werden.

4. Verfahren nach einem der Ansprüche 1 - 3, dadurch gekennzeichnet, daß die Lage der Bezugspunkte (7 - 10, 7' - 10'; 8'' - 10'') in wenigstens zwei Betrachtungsachsen erfaßt wird.

5. Verfahren nach einem der Ansprüche 1 - 3, dadurch gekennzeichnet, daß vier räumlich gegeneinander versetzte Bezugspunkte (7 - 10, 7' - 10'; 8'' - 10'') angebracht werden.

6. Verfahren nach einem der Ansprüche 1 - 5, dadurch gekennzeichnet, daß an wenigstens einem Kiefer (1, 2) wenigstens ein Befestigungselement (13, 14, 38, 39) befestigt wird, welche die räumlich gegeneinander versetzenden Bezugspunkte (7 - 10, 7' - 10'; 8'' - 10'') aufweist.

7. Verfahren nach einem der Ansprüche 1 - 6, dadurch gekennzeichnet, daß zur Bestimmung der Relativbewegung zwischen den Kiefern (1, 2) in einer Achsrichtung (H) zusätzlich ein Lichtstrahl verwendet wird, welcher von einer an einem Kiefer (1, 2) oder von einer an einem Bezugselement (13, 14) vorgesehenen Lichtquelle (17) ausgeht und an einer reflektierenden Fläche am anderen Kiefer (2, 1) bzw. an einem dort vorgesehenen Bezugselement (14, 13) reflektiert wird.

8. Verfahren nach einem der Ansprüche 1 - 7, dadurch gekennzeichnet, daß der von den Bezugspunkten (7 - 10, 7' - 10'; 8'' - 10'') gebildete Meßbereich mit zwei Videokameras (20, 25) unter unterschiedlichen Betrachtungswinkeln (A, A') betrachtet wird.

9. Verfahren nach einem der Ansprüche 1 - 7, dadurch gekennzeichnet, daß bei Verwendung einer Videokamera (20) mit dieser Kamera der von den Bezugspunkten (7 - 10, 7' - 10') gebildete Meßbereich über eine optische Umlenkeinrichtung, beispielsweise über eine Spiegeleinrichtung in zeitlicher Folge aus zwei Betrachtungsrichtungen (A, A') erfaßt wird.

10. Meßanordnung zur dreidimensionalen Bestimmung der Relativbewegung zwischen einem Unterkiefer und einem Oberkiefer, mit räumlich gegeneinander versetzten Bezugspunkten (7 - 10, 7' - 10'; 8'' - 10'') an den Kiefern (1, 2) sowie mit einer opto-elektrischen Einrichtung, die im Abstand von dem von den Bezugspunkten gebildete Meßbereich angeordnet und mit der die Lage der Bezugspunkte relativ zueinander optisch erfaßbar ist, dadurch gekennzeichnet, daß an beiden Kiefern (1, 2) jeweils wenigstens drei räumlich gegeneinander versetzte Bezugspunkte (7 - 10, 7' - 10'; 8'' - 10'') vorgesehen sind, und daß die opto-elektrische Einrichtung wenigstens eine Videokamera (20, 25) aufweist, mit der die Lage der Bezugspunkte an jedem Kiefer relativ zueinander sowie relativ zu den Bezugspunkten des jeweils anderen Kiefers erfaßbar ist.

11. Meßanordnung nach Anspruch 10, dadurch gekennzeichnet, daß jeweils vier Bezugspunkte (7 - 10, 7' -10'; 8'' -10'') räumlich gegeneinander versetzt vorgesehen sind.

12. Meßanordnung nach Anspruch 10 oder 11, dadurch gekennzeichnet, daß die Bezugspunkte (7 - 10, 7' - 10'; 8'' -10'') von den Eckpunkten oder von anderen markanten Punkten bzw. Bereichen eines zwei- oder dreidimensionalen Körpers (11, 12, 38, 39, 39' 39'') gebildet sind.

13. Meßanordnung nach Anspruch 12, dadurch gekennzeichnet, daß die Bezugspunkte (7 - 10, 7' - 10) von den Eckpunkten eines pyramidenartigen Körpers (11, 12) gebildet sind.

14. Meßanordnung nach einem der Ansprüche 10 - 13, dadurch gekennzeichnet, daß an wenigstens einem Kiefer (1, 2) mindestens eine Lichtquelle (17) vorgesehen ist, die einen gebündelten Lichtstrahl (18) aussendet, welcher an einer Reflexionsfläche, z.B. an einem Spiegel (19) am anderen Kiefer (2, 1) in Richtung zur opto-elektrischen Einrichtung reflektiert wird.

15. Meßanordnung nach einem der Ansprüche 10 - 14, dadurch gekennzeichnet, daß die Bezugspunkte (7 - 10, 7' - 10'; 8'' - 10'') als Licht-Reflexionsflächen ausgebildet und/oder durch Kontrast, Helligkeit und/oder Farbgebung optisch markant hervorgehoben und/oder als Lichtpunkte ausgebildet sind, die vorzugsweise zur Identifikation der einzelnen Bezugspunkte Licht mit unterschiedlicher Farbe und/oder ein in der Helligkeit moduliertes Lichtsignal gleicher oder unterschiedlicher Frequenz abgeben.

16. Meßanordnung nach einem der Ansprüche 10 - 15, dadurch gekennzeichnet, daß die Videokamera (20 - 25) mit einem Bildspeicher (21, 26) verbunden ist.

17. Meßanordnung nach einem der Ansprüche 10 - 16, dadurch gekennzeichnet, daß die opto-elektrische Einrichtung zwei Videokameras (20, 25) aufweist, die in unterschiedlicher Betrachtungsrichtung (A, A') auf den von den Bezugspunkten (7 - 10, 7' - 10') gebildete Meßbereich gerichtet sind.

18. Meßanordnung nach einem der Ansprüche 10 - 16, dadurch gekennzeichnet, daß die opto-elektrische Einrichtung eine Videokamera (20) aufweist, mit der über eine optische Umlenkeinrichtung, z.B. Spiegelanordnung, in zeitlicher Folge nacheinander der von den Bezugspunkten (7 - 10, 7' - 10'; 8'' - 10'') gebildete Meßbereich aus zwei unterschiedlichen Betrachtungsrichtungen erfaßt wird.

19. Meßanordnung nach einem der Ansprüche 10 - 18, dadurch gekennzeichnet, daß die opto-elektrische Einrichtung eine Datenverarbeitungseinheit (24) mit Datenspeicher (23) aufweist, in der aus der jeweiligen Lage der Bezugspunkte (7 - 10, 7' - 10'; 8'' - 10'') zueinander nach einem Programm die Relativbewegung zwischen den Kiefern (1, 2) ermittelt wird.

20. Meßanordnung nach einem der Ansprüche 10 - 19, dadurch gekennzeichnet, daß die opto-elektrische Einrichtung eine elektronische Abtasteinheit (22) aufweist, in welcher zur Ermittlung der Lage der Bezugspunkte (7 - 10, 7' - 10'; 8'' - 10'') die von der wenigstens einen Videokamera (20, 25) gelieferten Einzelbilder oder Teilbilder zeilen- oder spaltenmäßig nach dem Vorhandensein der Bezugspunkte abgetastet werden.

**Claims**

1. Method for the three dimensional determination of the relative movement of a lower and upper jaw in which reference points (7-10, 7'-10'; 8"-10") are fastened onto the jaws (1,2), their positions relative to one another being fixed at least in an optical axis direction, and in which the relative movement of the lower jaw (2) and upper jaw (1) is determined by the positional change, in respect of one another, of the reference points, whereby at any one time, at the lower jaw (2) and at the upper jaw (1) at least three spatial reference points (7-10, 7'-10'; 8"-10") are fixed, displaced with reference to each other, and that the relative position of the reference points (7-10, 7'-10'; 8"-10") is captured with the aid of at least a video camera (20, 25) and from this, by computer, the relative movement of the lower jaw and upper jaw is determined.

2. Method, as claimed in claim 1, whereby the position of the reference points (7-10, 7'-10'; 8"-10") relative to one another is captured with the aid of an image store (21) connected to the video camera.

3. Method, as claimed in claim 1 or 2, whereby the stored individual images supplied from the video camera (20, 25) and/or in the image store (21, 26) are scanned line - and column - wise for the determination of the position of the reference points (7-10, 7'-10'; 8"-10").

4. Method, as claimed in one of claims 1-3, whereby the position of the reference points (7-10, 7'-10'; 8"-10") is captured in at least two viewing axes.

5. Method, as claimed in one of claims 1-3, whereby four spatial reference points (7-10, 7'-10'; 8"-10") displaced with reference to each other, are fixed.

6. Method, as claimed in one of claims 1-5, whereby on at least one jaw (1, 2), at least one fastening element (13, 14, 38, 39) is fixed, which shows the spatial reference points (7-10, 7'-10'; 8"-10") moving with respect to each other.

7. Method, as claimed in one of claims 1-6, whereby to determine the relative movement between the jaws (1, 2) in an axis direction (H), a light beam is additionally used which comes from a light source (17) located on a jaw (1, 2) or on a reference element (13, 14) and is reflected on a reflective surface on the other jaw (2, 1) or on a reference element (14, 13) located there.

8. Method, as claimed in one of claims 1-7, whereby the measuring area formed from the reference points (7-10, 7'-10'; 8"-10") is examined by two cameras (20, 25) under different viewing angles (A, A').

9. Method, as claimed in one of claims 1-7, whereby by the use of a video camera (20), with this camera the measuring area formed from the reference points (7-10, 7'-10') is captured via an optical means for direction for example via a mirror device in time order from two viewing angle directions (A, A').

10. Measuring equipment for the three dimensional determination of the relative movement between a lower jaw and an upper jaw, with spatial reference points (7-10, 7'-10'; 8"-10") displaced with respect to each other, on the jaws (1, 2), as well as with opto-electrical equipment, arranged at a distance from the reference point formed measurement area and with which the position of the reference points relative to one another is optically captured, whereby on both jaws (1, 2) at any time at least three spatial reference points (7-10, 7'-10'; 8"-10") displaced with respect to each other are provided, and that the opto-electrical equipment comprises at least a video camera (20, 25), with which the relative position to one another of the reference points on each jaw is captured as well as relative to the reference points of the other jaw, at any time.

11. Measuring equipment as claimed in claim 10, whereby at any time four spatial reference points (7-10, 7'-10'; 8"-10"), displaced with respect to each other, are provided.

12. Measuring equipment as claimed in claim 10 or 11, whereby the reference points (7-10, 7'-10'; 8"-10") are formed from corner points or from other defined points or rather spheres of two or three dimensional bodies (11, 12, 38, 39, 39', 39").

13. Measuring equipment as claimed in claim 12, whereby the reference points (7-10, 7'-10') are formed from the corner points of pyramidal bodies (11, 12).

14. Measuring equipment as claimed in one of claims 10 - 13, whereby on at least one jaw (1, 2) at least one light source (17) is provided, that emits a concentrated light beam (18) which is reflected on a reflective surface, for example on a mirror (19) on the other jaw (2, 1)

15. Measuring equipment as claimed in one of claims 10-14, whereby the reference points (7-10, 7'-10'; 8"-10") are formed out of light reflecting surfaces and/or made prominent through contrast, brightness and/or optical colouring and/or formed out of points of light which preferably emit light of a different colour and/or a light signal modulated in brightness of the same or a different frequency in order to identify the individual reference points.

16. Measuring equipment as claimed in one of claims 10-15, whereby the video camera (20 - 25) is connected to an image store.

17. Measuring equipment as claimed in one of claims 10-16, whereby the opto-electrical equipment comprises two video cameras (20, 25) directed in different viewing directions (A, A') onto the measuring area formed from the reference points (7-10, 7'-10'; 8"-10").

18. Measuring equipment as claimed in one of claims 10-16 whereby the opto-electrical equipment comprises a video camera (20), with via an optical means for direction for example mirror equipment, the measuring area formed by the reference points (7-10, 7'-10'; 8"-10") is captured from two different angles in time order succession.

19. Measuring equipment as claimed in one of claims 10-18, whereby the opto-electrical equipment comprises a data processor (24) with a data store, in which, from the respective positions of the reference points (7-10, 7'-10'; 8"10;") to one another, via a program, the relative displacement between the jaws (1, 2) is determined.

20. Measuring equipment, as claimed in one of claims 10-19, whereby the opto-electrical equipment consists of an electronic scan unit (22), in which, for the determination of the position of the reference points (7-10, 7'-10'; 8"-10") via the presence of the reference points, at least an individual image or partial image supplied by the video camera (20, 25) is scanned line - or column - wise.

**Revendications**

1. Procédé pour la détermination tridimensionnelle du mouvement relatif entre une mâchoire inférieure et une mâchoire supérieure, des points de référence (7 - 10, 7' - 10'; 8'' - 10'') étant placés aux mâchoires (1, 2) dont la position de l'un relativement à l'autre est détectée ou saisie optiquement dans au moins une direction axiale et le mouvement relatif de la mâchoire inférieure (2) et de la mâchoire supérieure (1) étant déterminé à partir du changement de position des points de référence l'un par rapport à l'autre, caractérisé en ce qu'on place à la mâchoire inférieure (2) et la mâchoire supérieure (1) chaque fois au moins trois points de référence (7 - 10, 7 - 10'; 8'' - 10'') décalés dans l'espace l'un par rapport à l'autre, on enregistre ou détecte la position des points de référence (7 - 10, 7' - 10'; 8'' - 10'') l'un par rapport à l'autre au moyen d'au moins une caméra vidéo (20, 25) et on détermine à partir de ceci de manière assistée par ordinateur le mouvement relatif de la mâchoire inférieure et de la mâchoire supérieure.

2. Procédé selon la revendication 1 caractérisé en ce que la position des points de référence (7 - 10, 7' - 10'; 8'' - 10'') l'un par rapport à l'autre est saisie ou enregistrée au moyen d'une mémoire enregistreuse d'images (21) connectée à la caméra vidéo.

3. Procédé selon la revendication 1 ou 2 caractérisé en ce que les images individuelles fournies par la caméra vidéo (20, 25) et/ou mémorisées dans la mémoire enregistreuse d'images sont explorées ou balayées par lignes et par colonnes pour la détermination de la position des points de référence (7 - 10, 7' - 10'; 8'' - 10'').

4. Procédé selon l'une des revendications 1 à 3 caractérisé en ce que la position des points de référence (7 - 10, 7' - 10'; 8'' - 10'') est saisie ou détectée suivant au moins deux axes d'observation.

5. Procédé selon l'une des revendictions 1 à 3 caractérisé en ce que quatre points de référence décalés spatialement l'un par rapport à l'autre (7 - 10, 7' - 10'; 8'' - 10'') sont mis en place.

6. Procédé selon l'une des revendications 1 à 5 caractérisé en ce qu'on fixe sur au moins une mâchoire (1, 2) au moins un élément de fixation (13, 14, 38, 39) qui comporte les points de référence décalés spatialement l'un par rapport à l'autre (7 - 10, 7' - 10'; 8'' - 10'').

7. Procédé selon l'une des revendications 1 à 6 caractérisé en ce que pour la détermination du mouvement relatif entre les mâchoires (1, 2) dans une direction axiale (H), on utilise en supplément un rayon lumineux qui part d'une source lumineuse (17) prévue sur une mâchoire (1, 2) ou sur un élément de référence (13, 14) et qui est réfléchi sur une surface réfléchissante à l'autre mâchoire (2, 1) ou à un élément de référence (14, 13) prévu à cet emplacement.

8. Procédé selon l'une des revendications 1 à 7 caractérisé en ce que la zone de mesure formée par les points de référence (7 - 10, 7' - 10'; 8'' - 10'') est observée avec deux caméras vidéo (20, sous des angles d'observation (A, A') différents.

9. Procédé selon l'une des revendications 1 à 7 caractérisé en ce que lorsqu'on utilise une caméra vidéo (20), on enregistre ou saisit avec cette caméra la zone de mesure formée par les points de référence (7 - 10, 7' - 10'; 8'' - 10'') au moyen d'une installation de déviation optique, par exemple d'une installation à miroirs, de manière successive temporelle à partir de deux directions d'observation (A, A').

10. Arrangement de mesure pour la détermination tridimensionnelle du mouvement relatif entre une mâchoire inférieure et une mâchoire supérieure avec des points de référence (7 - 10, 7' - 10'; 8'' - 10'') décalés spatialement l'un par rapport à l'autre aux mâchoires (1, 2) ainsi qu'avec une installation opto-électrique qui est disposée à distance de la zone de mesure formée par les points de référence et avec laquelle la position des points de référence l'un relativement à l'autre est enregistrable ou saisissable optiquement, caractérisé en ce qu'on prévoit aux deux mâchoires (1, 2) chaque fois au moins trois points de référence (7 - 10, 7' - 10'; 8'' - 10'') décalés spatialement l'un par rapport à l'autre et l'installation opto-électrique comporte au moins une caméra vidéo (20, 25) avec laquelle la position des points de référence à chaque mâchoire l'un relativement à l'autre ainsi que relativement aux points de référence de l'autre mâchoire est enregistrable.

11. Arrangement de mesure selon la revendication 10 caractérisé en ce que l'on prévoit chaque fois quatre points de référence (7 - 10, 7' - 10'; 8'' - 10'') décalés spatialement l'un par rapport à l'autre.

12. Arrangement de mesure selon la revendication 10 ou 11 caractérisé en ce que les points de référence (7 - 10, 7' - 10'; 8'' - 10'') sont formés par les points correspondant aux coins ou aux sommets ou par d'autres points au régions marquants d'un corps bidimensionnel ou tridimensionnel (11, 12, 38, 39, 39', 39'').

13. Arrangement de mesure selon la revendication 12 caractérisé en ce que les points de référence (7 - 10, 7' - 10) sont formés par les sommets d'un corps pyramidal (11, 12).

14. Arrangement de mesure selon l'une des revendications 10 à 13 caractérisé en ce qu'on prévoit à au moins une mâchoire (1, 2) au moins une source lumineuse (17) qui émet un faisceau lumineux (18) lequel est réfléchi sur une surface de réflexion, par exemple sur un miroir (19) à l'autre mâchoire (2, 1) en direction de l'installation opto-électrique.

15. Arrangement de mesure selon l'une des revendications 10 à 14 caractérisé en ce que les points de référence (7 - 10, 7' - 10'; 8'' - 10'') sont réalisés sous forme de surfaces de réflexion de lumière et/ou sont mis en évidence de manière à être optiquement marquants par contraste, clarté et/ou couleur et/ou sont réalisés sous forme de points lumineux qui émettent de préférence pour l'identification des points de référence individuels de la lumière de couleur différente et/ou un signal lumineux modulé en luminosité de fréquence identique ou différente.

16. Arrangement de mesure selon l'une des revendications 10 à 15 caractérisé en ce la caméra vidéo (20, 25) est connectée à une mémoire enregistreuse d'images 21, 26).

17. Arrangement de mesure selon l'une des revendications 10 à 16 caractérise en ce que l'installation opto-électrique comporte deux caméras vidéo (20, 25) qui sont dirigées suivant des directions d'observation différentes (A, A') vers la zone de mesure formée par les points de référence (7 - 10, 7' - 10').

18. Arrangement de mesure selon l'une des revendications 10 à 16 caractérisé en ce que l'installation opto-électrique comporte une caméra vidéo (20) avec laquelle on enregistre au moyen d'une installation de déviation optique, par exemple un arrangement à miroirs, successivement dans le temps la zone de mesure formée par les points de référence (7 - 10, 7' - 10'; 8'' - 10'') à partir de deux directions d'observation différentes.

19. Arrangement de mesure selon l'une des revendications 10 à 18 caractérisé en ce que l'installation opto-électrique comporte une unité de traitement de données (24) à mémoire de données (23), dans laquelle on détermine le mouvement relatif entre les mâchoires (1, 2) suivant un programme à partir de chacune des positions des points de référence (7 - 10, 7' - 10'; 8'' - 10'') l'un par rapport à l'autre.

20. Arrangement de mesure selon l'une des revendications 10 à 19 caractérisé en ce que l'installation opto-électrique comporte une unité de balayage électronique (22) dans laquelle pour la détermination de la position des points de référence (7 - 10, 7' - 10'; 8'' - 10'') les images individuelles ou partielles fournies par la caméra vidéo (20, 25) (au moins une) sont balayées ou explorées par lignes ou par colonnes pour rechercher la présence des points de référence.

Fig.1

Fig.2

Fig.3

Fig.4

EP 0 263 316 B1

Fig.5

Fig.6

EP 0 263 316 B1

Fig 7

Fig 8

46

44

45

39'

10"(9")

Fig 9

8'

10'(9')

8"

39"